# EUROPEAN PATENT SPECIFICATION

(11) **EP 4 067 366 B1**
(45) Date of publication and mention of the grant of the patent: **21.10.2026**
(21) Application number: 21813135.7
(22) Date of filing: 25.02.2021
(51) Int. Cl.: C07J 7/00, C07J 9/00, C07J 31/00

(54) **METHOD FOR PURIFYING PROGESTERONE**
VERFAHREN ZUR REINIGUNG VON PROGESTERON
PROCÉDÉ DE PURIFICATION DE LA PROGESTÉRONE

(30) Priority: 25.05.2020 CN 202010451197
(43) Date of publication of application: 05.10.2022
(73) Proprietor: Zhejiang Shenzhou Pharmaceutical Company Limited, Chengguan Town, Xianju County Taizhou Zhejiang 317300 (CN)
(72) Inventor: SHAO, Zhenping, Zhejiang 317300 (CN); WANG, Rong, Zhejiang 317300 (CN); WANG, Bingqian, Zhejiang 317300 (CN); WANG, Hongfu, Zhejiang 317300 (CN); HUANG, Chengcheng, Zhejiang 317300 (CN); LEI, Lingzhi, Zhejiang 317300 (CN); WANG, Youfu, Zhejiang 317300 (CN)
(74) Representative: Long, Giorgio
(86) International application number: PCT/CN2021/077911
(87) International publication number: WO 2021/238312

(56) References cited:
- CN-A- 110 204 585
- CN-A- 110 563 790
- CN-A- 112 062 801
- CN-A- 112 110 971
- CN-A- 112 250 727
- CN-A- 112 390 841
- FR-A- 1 082 645
- US-A- 4 201 716
- US-A- 4 222 949
- MARIA M. BOUCHER ET AL: "Liquid–Liquid Extraction Protocol for the Removal of Aldehydes and Highly Reactive Ketones from Mixtures", ORGANIC PROCESS RESEARCH & DEVELOPMENT, vol. 21, no. 9, 21 July 2017 (2017-07-21), US, pages 1394 - 1403, XP055525826, ISSN: 1083-6160, DOI: 10.1021/acs.oprd.7b00231
- HOU HAIBO, YANQING YANG, JIANXIONG ZHENG: "Study on the Purification Techniques of Mother Liquid of Progesterone", GUANGDONG CHEMICAL INDUSTRY, vol. 46, no. 2, 28 February 2019 (2019-02-28), pages 104 - 105, XP055873290
- TROST BARRY M., TAMARU YOSHINAO: "New synthetic reactions. Oxidative decarboxylation of .alpha.-methylthiocarboxylic acids, new approach to acyl anion and ketene synthons", JOURNAL OF THE AMERICAN CHEMICAL SOCIETY, AMERICAN CHEMICAL SOCIETY, vol. 99, no. 9, 1 April 1977 (1977-04-01), pages 3101 - 3113, XP055871868, ISSN: 0002-7863, DOI: 10.1021/ja00451a041
- "Sodium Formaldehyde Bisulfite", BAIDU BAIKE, CN, pages 1 - 3, XP009532335, Retrieved from the Internet <URL:https://baike.baidu.com/item/%E7%94%B2%E9%86%9B%E4%BA%9A%E7%A1%AB%E9%85%B8%E6%B0%A2%E9%92%A0/7613326>

## Description

### TECHNICAL FIELD

The present disclosure relates to the technical field of preparation process of steroids, specifically to a purification method of progesterone, and in particular to a purification method of a progesterone crude product containing aldehyde impurities.

### BACKGROUND ART

Progesterone is a natural progesterone secreted by the corpus luteum of the ovary, which has a significant morphological effect on the endometrium excited by estrogen, is necessary for pregnancy, and plays an important role in clinical medicine.

Using bisnoralcohol as a starting material, there are a plurality of methods for synthesizing progesterone.

Method A is to oxidize bisnoralcohol into bisnoraldehyde, followed by catalytic oxidation to yield progesterone. Its synthetic route is as follows:

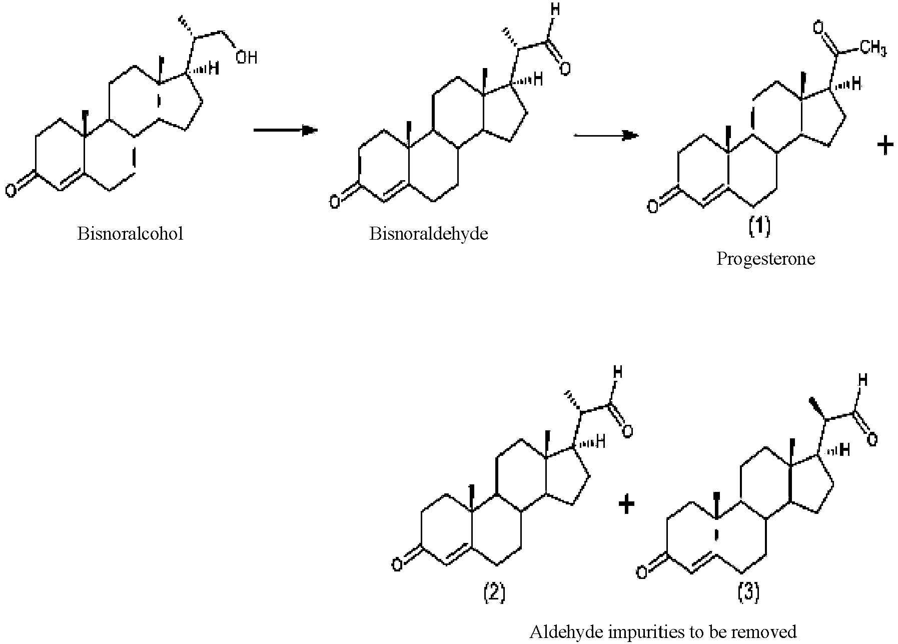

This method is difficult to convert bisnoraldehyde completely during the catalytic oxidation. Therefore, there are a plurality of bisnoraldehyde impurity (2) and its C20 epimer impurity (3) in final product progesterone.

In Method B, bisnoralcohol is oxidized into bisnoraldehyde, the bisnoraldehyde is further reacted to produce enamine, and the enamine is catalytically oxidized to yield progesterone. Enamine intermediate in this method is also difficult to react completely, which will also be converted into bisnoraldehyde impurity (which also contains its C20 epimer). Its synthetic route is as follows:

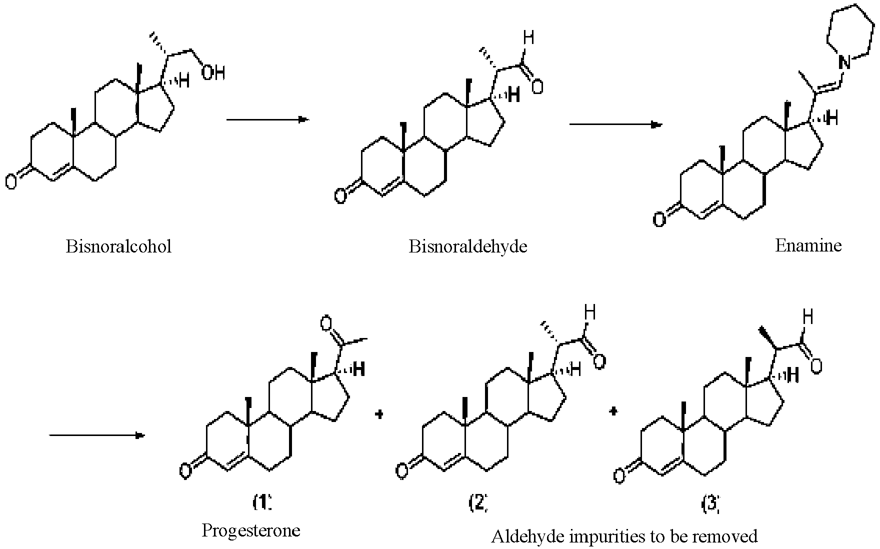

Visibly, if bisnoralcohol is used as a starting material to synthesize progesterone, a plurality of bisnoraldehyde impurities will be present in the final product. Furthermore, because bisnoraldehyde impurities have physicochemical properties close to progesterone, it is difficult to or there is no way to remove them by using conventional purification methods such as recrystallization and adsorption, posing an insurmountable barrier to obtaining highly pure progesterone in industrial production. It has been tried to use a chromium-containing oxidant to oxidize bisnoraldehyde into an acid and remove aldehyde impurities by alkaline wash. However, this method is cumbersome in operation, produces a great deal of wastewater containing chromium; moreover, oxidization with oxidants may bring some new organic impurities and heavy metal impurities, which increases the difficulty of purification and is not suitable for industrial production.

As described in European Pharmacopoeia 9.0, the content of bisnoraldehydes (2) and (3) in progesterone substance on HPLC must be <0.6%, otherwise it is considered as an unqualified product. The content of bisnoraldehydes in progesterone crude product is 2 to 3%. Through general purification methods, there is no way to reduce the content of the impurity to 0.6% or below, namely, it is difficult to produce qualified progesterone substance that complies with the European Pharmacopoeia.

FR1082645A discloses preparing crude progesterone by dissolving the crude product mixture in a mixed solvent of ether and methanol, adding sodium bisulphite thereto, and extracting with ether, thus obtaining a crude product. Pure progesterone is obtained by recrystallization from

### ether.

### SUMMARY

To solve the above problems present in the prior art, the present disclosure avoids the technical bottleneck of progesterone purification in the prior art, and provides a purification method to obtain highly pure progesterone, which is simple to operate and has a significant effect.

The invention is set out in the appended claim.

In the method provided by the present disclosure, the weight is in gram (g), and the volume is in milliliter (mL).

All raw materials related in the method provided by the present disclosure may be commercially available.

Compared with the prior art, the present disclosure has the following beneficial effects:
1) The purification method provided by the present disclosure avoids the technical bottleneck of progesterone purification in the prior art, operations are simple and convenient, with low equipment requirement and cost input;
2) in the present disclosure, purified progesterone content is higher than 99.5%, total mass yield is higher than 93%, and total aldehyde impurities content is lower than 0.2%, which meets the requirement as described in the European Pharmacopoeia 9.0, namely, the content of bisnoraldehydes (2) and (3) in progesterone substance on HPLC must be <0.6%;
3) the purification process in the present disclosure is mild and safe, conditions are easy to control, and reagents for removing aldehyde impurities are inexpensive and easily available and have very high market competitiveness in cost;
4) reagents used in the purification method provided by the present disclosure cause little environmental pollution, and particularly highly polluting chromium-containing reagents are not used, coinciding with the development trend of the green chemical industry and having excellent economic and social benefits.

### BRIEF DESCRIPTION OF THE DRAWINGS

FIG. 1 is a high performance liquid chromatogram of a progesterone crude product.
FIG. 2 is a high performance liquid chromatogram of a finished product of progesterone obtained by the purification method provided by the present disclosure.
FIG. 3 is a high performance liquid chromatogram of progesterone obtained by a common purification method.

### DETAILED DESCRIPTION OF THE EMBODIMENTS

Specific experimental procedures or conditions that are not indicated in the present disclosure shall be implemented by operations of conventional experimental procedures described in the disclosure in the art. All used reagents or equipment without specifying manufacturers may be commercially available conventional products.

### Example 1 A purification method of highly pure progesterone (according to the invention)

50 g of progesterone crude product (containing 2% aldehyde impurities) was fed into 50 mL of ethanol, completely dissolved, stirred with 2.5 mL of 40% aqueous sodium metabisulfite solution at 60°C to react for 1 h, and cooled down to 5°C to obtain an initial reaction treated product.

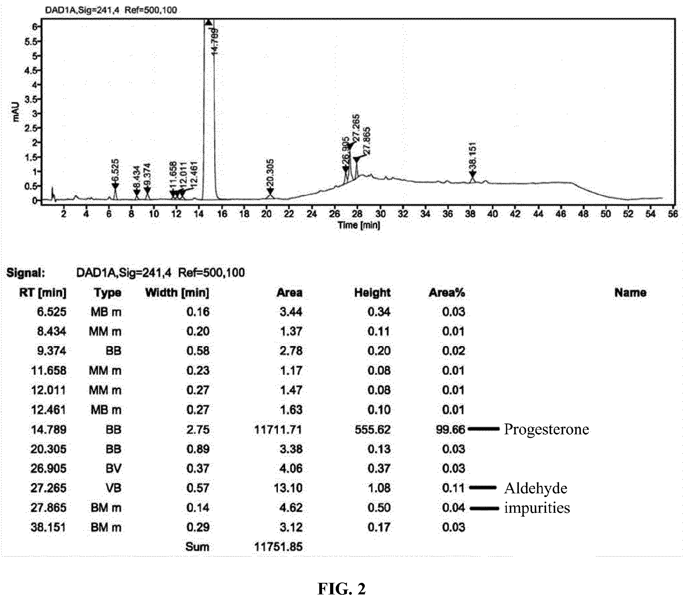

The initial reaction treated product was supplemented with 25 mL of water; after filtration, the filter cake was washed with water until neutral and dried to obtain 47.8 g of finished product of progesterone. The product yield was 95.6%, the melting point of the product was 129.0 to 130.0°C, the content on HPLC was 99.6%, and the aldehyde impurities content was <0.15%. The high performance liquid chromatogram of the finished product of progesterone is shown in FIG. 2.

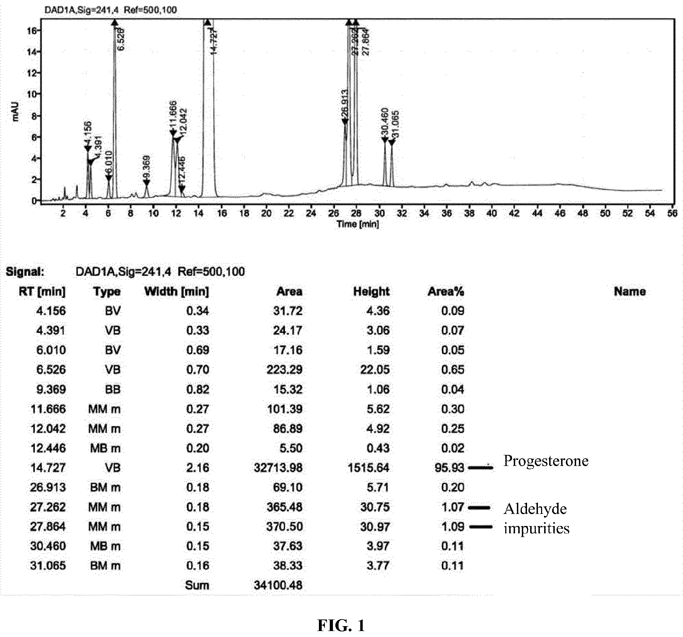

The high performance liquid chromatogram of the progesterone crude product is shown in FIG. 1.

### Example 2 A purification method of highly pure progesterone (according to the invention)

50 g of progesterone crude product (containing 3% aldehyde impurities) was fed into 350 mL of isopropanol, completely dissolved, stirred with 25 mL of 20% aqueous potassium bisulfite solution at 40°C to react for 0.1 h, and cooled down to -5°C to obtain an initial reaction treated product.

The initial reaction treated product was supplemented with 150 mL of water; after filtration, the filter cake was washed with water until neutral and dried to obtain 47.6 g of finished product of progesterone. The product yield was 95.2%, the melting point of the product was 129.1 to 130.0°C, the content on HPLC was 99.5%, and the aldehyde impurities content was <0.2%.

### Example 3 A purification method of highly pure progesterone (according to the invention)

50 g of progesterone crude product (containing 3% aldehyde impurities) was fed into 750 mL of tert-butanol, completely dissolved, stirred with 50 mL of 15% aqueous potassium metabisulfite solution at 20°C to react for 2 h, and cooled down to -20°C to obtain an initial reaction treated product.

The initial reaction treated product was supplemented with 250 mL of water; after filtration, the filter cake was washed with water until neutral and dried to obtain 47.5 g of finished product of progesterone. The product yield was 95.0%, the melting point of the product was 129.1 to 130.2°C, the content on HPLC was 99.6%, and the aldehyde impurities content was <0.15%.

### Example 4 A purification method of highly pure progesterone (according to the invention)

50 g of progesterone crude product (containing 3% aldehyde impurities) was fed into 100 mL of ethanol, completely dissolved, stirred with 15 mL of 35% aqueous sodium bisulfite solution at 50°C to react for 0.5 h, and cooled down to -10°C to obtain an initial reaction treated product.

The initial reaction treated product was supplemented with 100 mL of drinking water; after filtration, the filter cake was washed with water until neutral and dried to obtain 47.6 g of finished product of progesterone. The product yield was 95.2%, the melting point of the product was 129.0 to 130.0°C, the content on HPLC was 99.7%, and the aldehyde impurities content was <0.15%.

### Example 5 A purification method of highly pure progesterone (comparative example)

50 g of progesterone crude product (containing 2% aldehyde impurities) was fed into 50 mL of dichloromethane and 25 mL of ethanol, completely dissolved, intensively stirred with 25 mL of 50% aqueous sodium bisulfite solution at 30°C to react for 1 h, extracted with 250 mL of dichloromethane, separated, washed with water until neutral, and concentrated to obtain an initial reaction treated product.

The initial reaction treated product was supplemented with 25 mL of water; after filtration, the filter cake was washed with water until neutral and dried to obtain 47.0 g of finished product of progesterone. The product yield was 94.0%, the melting point of the product was 128.8 to 130.0°C, the content on HPLC was 99.5%, and the aldehyde impurities content was <0.2%.

### Example 6 A purification method of highly pure progesterone (comparative example)

50 g of progesterone crude product (containing 3% aldehyde impurities) was fed into 250 mL of dichloroethane and 250 mL of methanol, completely dissolved, intensively stirred with 250 mL of 5% aqueous sodium metabisulfite solution at 20°C to react for 2 h, extracted with 1,500 mL of dichloroethane, separated, washed with water until neutral, and concentrated to obtain an initial reaction treated product.

The initial reaction treated product was supplemented with 250 mL of water; after filtration, the filter cake was washed with water until neutral and dried to obtain 46.8 g of finished product of progesterone. The product yield was 93.6%, the melting point of the product was 128.9 to 130.0°C, the content on HPLC was 99.6%, and the aldehyde impurities content was <0.2%.

### Example 7 A purification method of highly pure progesterone (comparative example)

50 g of progesterone crude product (containing 3% aldehyde impurities) was fed into 100 mL of toluene and 200 mL of tert-butanol, completely dissolved, intensively stirred with 100 mL of 30% aqueous potassium bisulfite solution at 60°C to react for 0.1 h, extracted with 750 mL of toluene, separated, washed with water until neutral, and concentrated to obtain an initial reaction treated product.

The initial reaction treated product was supplemented with 100 mL of water; after filtration, the filter cake was washed with water until neutral and dried to obtain 46.5 g of finished product of progesterone. The product yield was 93.0%, the melting point of the product was 128.5 to 129.8°C, the content on HPLC was 99.5%, and the aldehyde impurities content was <0.2%.

### Example 8 A purification method of highly pure progesterone (comparative example)

25 mL of 50% aqueous sodium sulfite solution was added into 50 mL of ethanol, and SO₂ was passed until pH = 6; 50 g of progesterone crude product (containing 2% aldehyde impurities) was added, completely dissolved, stirred at 60°C to react for 0.1 h, and cooled down to 5°C to obtain an initial reaction treated product.

The initial reaction treated product was supplemented with 25 mL of water; after filtration, the filter cake was washed with water until neutral and dried to obtain 47.0 g of finished product of progesterone. The product yield was 94.0%, the melting point of the product was 128.9 to 129.8°C, the content on HPLC was 99.6%, and the aldehyde impurities content was <0.15%.

### Example 9 A purification method of highly pure progesterone (comparative example)

50 mL of 10% aqueous sodium hydroxide solution was added into 300 mL of tert-butanol, and SO₂ was passed until pH = 5; 50 g of progesterone crude product (containing 3% aldehyde impurities) was added, completely dissolved, stirred at 30°C to react for 1 h, and cooled down to -5°C to obtain an initial reaction treated product.

The initial reaction treated product was supplemented with 150 mL of water; after filtration, the filter cake was washed with water until neutral and dried to obtain 46.8 g of finished product of progesterone. The product yield was 94.0%, the melting point of the product was 128.9 to 129.2°C, the content on HPLC was 99.5%, and the aldehyde impurities content was <0.15%.

### Example 10 A purification method of highly pure progesterone (comparative example)

250 mL of 5% aqueous potassium hydroxide solution was added into 750 mL of methanol, and SO₂ was passed until pH = 5; 50 g of progesterone crude product (containing 2% aldehyde impurities) was added, completely dissolved, stirred at 20°C to react for 2 h, and cooled down to -20°C to obtain an initial reaction treated product.

The initial reaction treated product was supplemented with 250 mL of water; after filtration, the filter cake was washed with water until neutral and dried to obtain 46.9 g of finished product of progesterone. The product yield was 93.8%, the melting point of the product was 128.8 to 129.2°C, the content on HPLC was 99.5%, and the aldehyde impurities content was <0.15%.

### Comparative Example 1 A conventional crystallization purification method of progesterone

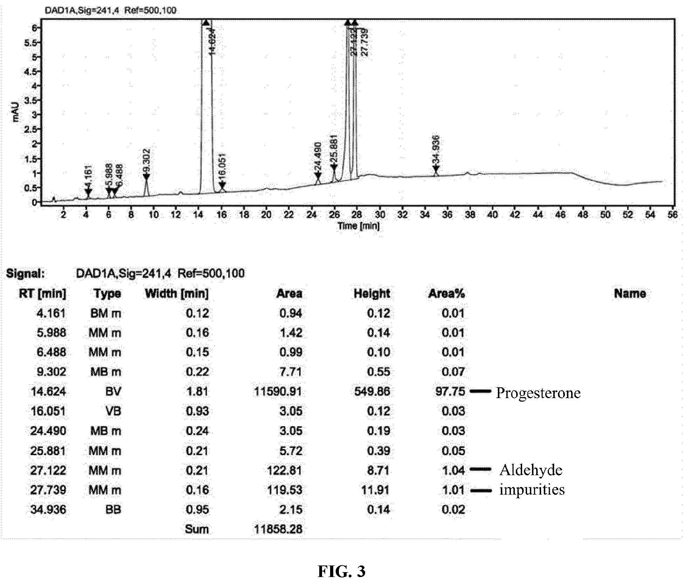

50 g of progesterone crude product (containing 2% aldehyde impurities) was added into 100 mL of ethanol, heated to 70°C for dissolution, stirred to cool down to 0°C, filtered and dried to obtain 46 g of ethanol-crystallized substance of progesterone. The purification yield was 92.0%, the content on HPLC was 97.7%, and the aldehyde impurities content was 2%. The high performance liquid chromatogram of the resulting product progesterone is shown in FIG. 3.

Conclusion: The conventional crystallization purification method has hardly any impact on reducing the aldehyde impurities content in the progesterone crude product, and there is no way to achieve the objective of the removal of aldehyde impurities even though the method is repeated many times.

### Comparative Example 2 A conventional oxidation and purification method of progesterone (chromic anhydride method)

50 g of progesterone crude product (containing 2% aldehyde impurities) was added into 150 mL of acetone, stirred with 10 mL of 10% aqueous CrO₃ solution at 25°C for 2 h, supplemented with 1,000 mL of water, and extracted with 200 mL of dichloromethane; the organic layer was washed once with 50 mL of 10% aqueous sodium hydroxide solution and with water until neutral, concentrated to remove dichloromethane, supplemented with 200 mL of water, cooled down to 5°C, filtered, and dried to obtain 45.0 g of progesterone. The purification yield was 90%, the content on HPLC was 95.0%, and the aldehyde impurities content was 0.8%.

Conclusion: The conventional purification method of progesterone by chromic anhydride oxidation can remove a part of aldehyde impurities from the progesterone crude product, but intensive reaction process may lead to an increase in other impurities, as well as heavy metal residues and pollution. Therefore, this method does not increase but decreases the purity of progesterone.

## Claims

1. A method for purifying progesterone, wherein the method has the following reaction route: 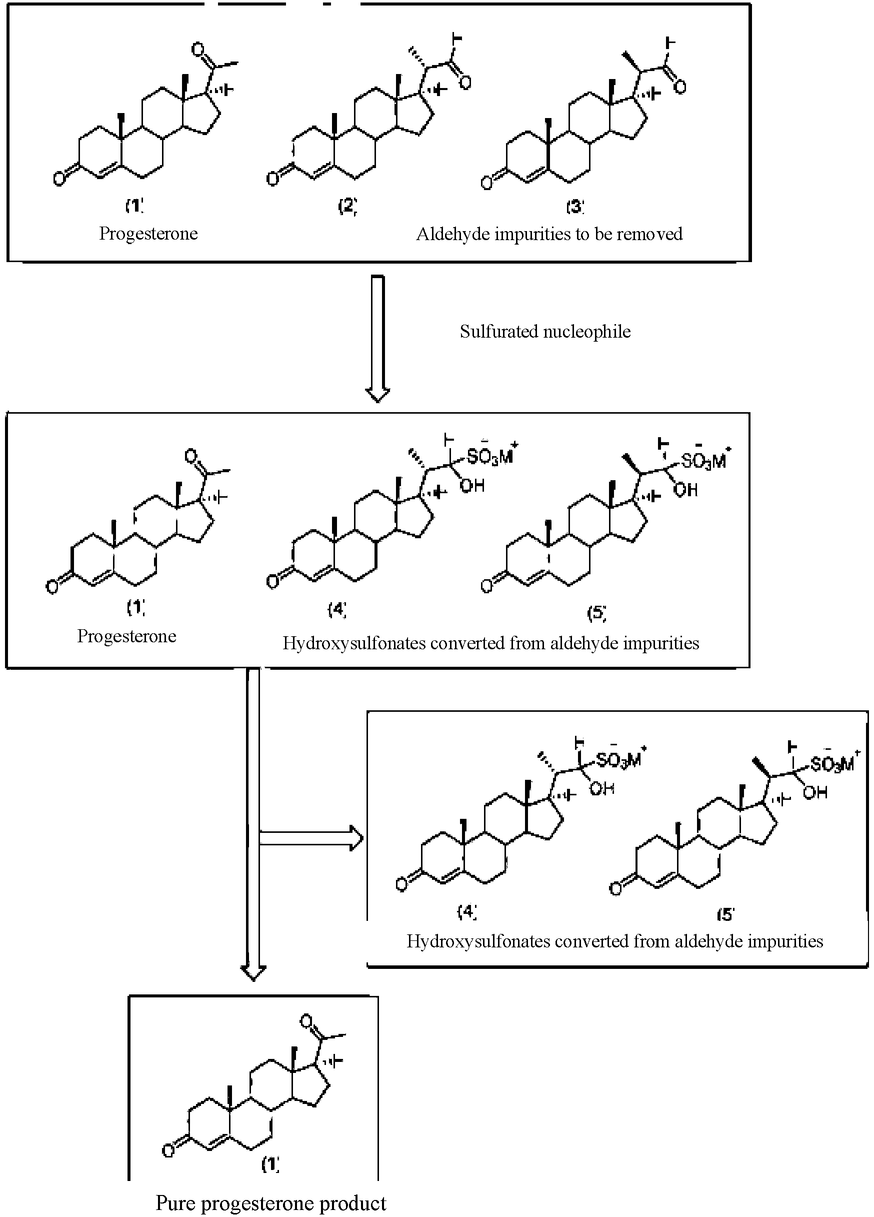 wherein M represents any one or more from the group consisting of Na, and K; and
the method consists of
feeding 50 g of a progesterone crude product containing 2% aldehyde impurities into 50 mL of ethanol until complete dissolution, stirring with 2.5 mL of 40% aqueous sodium metabisulfite solution at 60 °C to react for 1 h, and cooling down to 5 °C to obtain an initial reaction treated product; and
supplementing 25 mL of water to the initial reaction treated product, filtering, washing a resulting filter cake with water until neutral and drying to obtain a finished product of progesterone;
alternatively,
feeding 50 g of a progesterone crude product containing 3% aldehyde impurities into 350 mL of isopropanol until complete dissolution, stirring with 25 mL of 20% aqueous potassium bisulfite solution at 40 °C to react for 0.1 h, and cooling down to - 5°C to obtain an initial reaction treated product; and
supplementing 150 mL of water to the initial reaction treated product, filtering, washing a resulting filter cake with water until neutral and drying to obtain a finished product of progesterone;
alternatively,
feeding 50 g of a progesterone crude product containing 3% aldehyde impurities into 750 mL of tert-butanol until complete dissolution, stirring with 50 mL of 15% aqueous potassium metabisulfite solution at 20 °C to react for 2 h, and cooling down to -20 °C to obtain an initial reaction treated product; and
supplementing 250 mL of water to the initial reaction treated product, filtering, washing a resulting filter cake with water until neutral and drying to obtain a finished product of progesterone;
alternatively,
feeding 50 g of a progesterone crude product containing 3% aldehyde impurities into 100 mL of ethanol until complete dissolution, stirring with 15 mL of 35% aqueous sodium bisulfite solution at 50 °C to react for 0.5 h, and cooling down to -10 °C to obtain an initial reaction treated product; and
supplementing 100 mL of drinking water to the initial reaction treated product; filtering, washing a resulting filter cake with water until neutral and drying to obtain a finished product of progesterone.

## Patentansprüche

1. Verfahren zur Reinigung von Progesteron, wobei das Verfahren den folgenden Reaktionsweg aufweist: 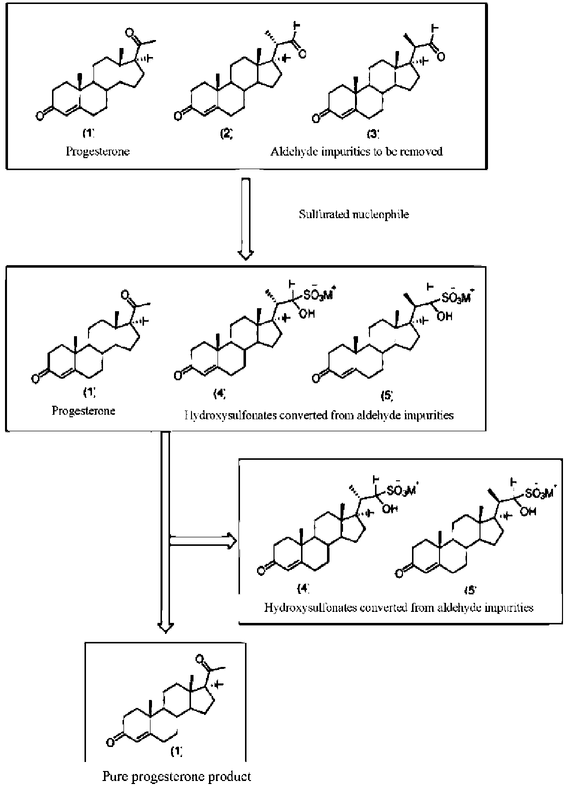
["Aldehyde impurities to be removed" = Zu entfernende Aldehydverunreinigungen;
"Sulfurated nucleophile" = Schwefelhaltiges Nukleophil;
"Hydroxysulfonates converted from aldehyde impurities" = Aus Aldehydverunreinigungen umgewandelte Hydroxysulfonate;
"Pure progesterone product" = Reines Progesteronprodukt]
wobei M für eines oder mehrere aus der Gruppe, bestehend aus Na und K, steht; und
wobei das Verfahren besteht aus Zuführen von 50 g eines Progesteron-Rohprodukts, das 2 % Aldehydverunreinigungen enthält, zu 50 ml Ethanol bis zur vollständigen Auflösung, Rühren mit 2,5 ml einer 40%igen wässrigen Natriummetabisulfitlösung bei 60 °C zur Reaktion über 1 h und Abkühlen auf 5 °C, um ein erstes reaktionsbehandeltes Produkt zu erhalten; und
Versetzen des ersten reaktionsbehandelten Produkts mit 25 ml Wasser, Filtrieren, Waschen eines resultierenden Filterkuchens mit Wasser bis zur Neutralität und Trocknen, um ein Progesteron-Fertigprodukt zu erhalten;
alternativ,
Zuführen von 50 g eines Progesteron-Rohprodukts, das 3 % Aldehydverunreinigungen enthält, zu 350 ml Isopropanol bis zur vollständigen Auflösung, Rühren mit 25 ml einer 20%igen wässrigen Kaliumbisulfitlösung bei 40 °C zur Reaktion über 0,1 h und Abkühlen auf -5 °C, um ein erstes reaktionsbehandeltes Produkt zu erhalten; und
Versetzen des ersten reaktionsbehandelten Produkts mit 150 ml Wasser, Filtrieren, Waschen eines resultierenden Filterkuchens mit Wasser bis zur Neutralität und Trocknen, um ein Progesteron-Fertigprodukt zu erhalten;
alternativ,
Zuführen von 50 g eines Progesteron-Rohprodukts, das 3 % Aldehydverunreinigungen enthält, zu 750 ml tert-Butanol bis zur vollständigen Auflösung, Rühren mit 50 ml einer 15%igen wässrigen Kaliummetabisulfitlösung bei 20 °C zur Reaktion über 2 h und Abkühlen auf -20 °C, um ein erstes reaktionsbehandeltes Produkt zu erhalten; und
Versetzen des ersten reaktionsbehandelten Produkts mit 250 ml Wasser, Filtrieren, Waschen eines resultierenden Filterkuchens mit Wasser bis zur Neutralität und Trocknen, um ein Progesteron-Fertigprodukt zu erhalten;
alternativ,
Zuführen von 50 g eines Progesteron-Rohprodukts, das 3 % Aldehydverunreinigungen enthält, zu 100 ml Ethanol bis zur vollständigen Auflösung, Rühren mit 15 ml einer 35%igen wässrigen Natriumbisulfitlösung bei 50 °C zur Reaktion über 0,5 h und Abkühlen auf -10 °C, um ein erstes reaktionsbehandeltes Produkt zu erhalten; und
Versetzen des ersten reaktionsbehandelten Produkts mit 100 ml Trinkwasser; Filtrieren, Waschen eines resultierenden Filterkuchens mit Wasser bis zur Neutralität und Trocknen, um ein Progesteron-Fertigprodukt zu erhalten.

## Revendications

1. Procédé de purification de progestérone, dans lequel le procédé présente la voie de réaction suivante : 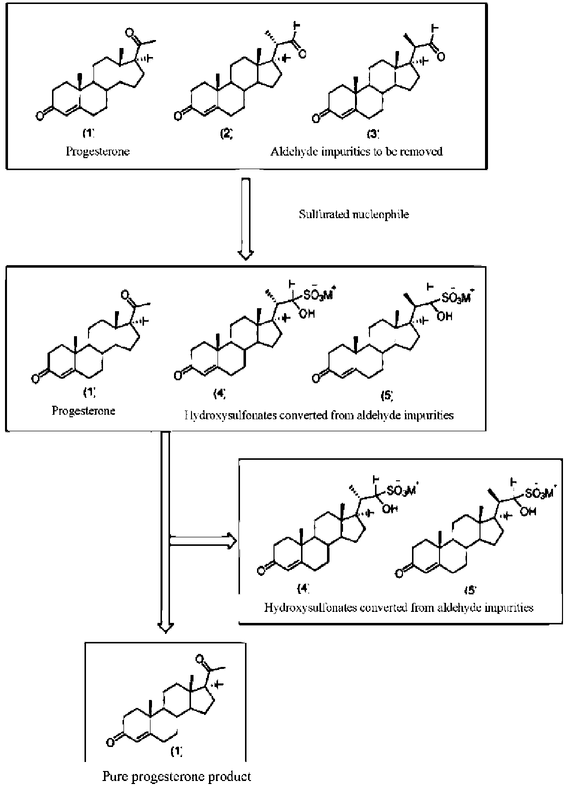
["Aldehyde impurities to be removed" = Impuretés d'aldéhyde à éliminer ;
"Sulfurated nucleophile" = Nucléophile sulfuré ;
"Hydroxysulfonates converted from aldehyde impurities" = Hydroxysulfonates convertis à partir d'impuretés d'aldéhyde ;
"Pure progesterone product" = Produit à base de progestérone pure]
dans lequel M représente un ou plusieurs éléments du groupe constitué de Na et K ; et
le procédé consiste à
introduire 50 g d'un produit brut de progestérone contenant 2% d'impuretés d'aldéhyde dans 50 mL d'éthanol jusqu'à dissolution complète, en agitant avec 2,5 mL de solution aqueuse de métabisulfite de sodium à 40 % à 60 °C pour réagir pendant 1 h, et en refroidissant à 5 °C pour obtenir un produit traité de réaction initiale ; et
ajouter 25 ml d'eau au produit traité de réaction initiale, filtrer, laver un gâteau de filtration résultant avec de l'eau jusqu'à neutralité et sécher pour obtenir un produit fini de progestérone ;
alternativement,
introduire 50 g d'un produit brut de progestérone contenant 3 % d'impuretés d'aldéhyde dans 350 mL d'isopropanol jusqu'à dissolution complète, agiter avec 25 mL de solution aqueuse de bisulfite de potassium à 20 % à 40 °C pour faire réagir pendant 0,1 h, et refroidir à - 5 °C pour obtenir un produit traité de réaction initiale ; et
ajouter 150 ml d'eau au produit traité de réaction initiale, filtrer, laver un gâteau de filtration résultant avec de l'eau jusqu'à neutralité et sécher pour obtenir un produit fini de progestérone ;
alternativement,
introduire 50 g d'un produit brut de progestérone contenant 3 % d'impuretés d'aldéhyde dans 750 mL de tert-butanol jusqu'à dissolution complète, agiter avec 50 mL de solution aqueuse de métabisulfite de potassium à 15 % à 20 °C pour réagir pendant 2 h, et refroidir à -20 °C pour obtenir un produit traité de réaction initiale ; et
ajouter 250 ml d'eau au produit traité de réaction initiale, filtrer, laver un gâteau de filtration résultant avec de l'eau jusqu'à neutralité et sécher pour obtenir un produit fini de progestérone ;
alternativement,
introduire 50 g d'un produit brut de progestérone contenant 3 % d'impuretés d'aldéhyde dans 100 mL d'éthanol jusqu'à dissolution complète, agiter avec 15 mL de solution aqueuse de bisulfite de sodium à 35 % à 50 °C pour réagir pendant 0,5 h, et refroidir à -10 °C pour obtenir un produit traité de réaction initiale ; et
ajouter 100 ml d'eau de boisson au produit traité de réaction initiale, filtrer, laver un gâteau de filtration résultant avec de l'eau jusqu'à neutralité et sécher pour obtenir un produit fini de progestérone.
